**Europäisches Patentamt**

**European Patent Office**

**Office européen des brevets**

(19)

(11) Veröffentlichungsnummer: **0 006 193**
A1

(12) # EUROPÄISCHE PATENTANMELDUNG

(21) Anmeldenummer: 79101811.2

(22) Anmeldetag: 07.06.79

(51) Int. Cl.³: **A 61 B 10/00,** G 01 S 15/00, G 01 N 29/00

(30) Priorität: 15.06.78 DE 2826277

(43) Veröffentlichungstag der Anmeldung: 09.01.80 Patentblatt 80/1

(84) Benannte Vertragsstaaten: **AT CH FR GB NL**

(71) Anmelder: **SIEMENS AKTIENGESELLSCHAFT Berlin und München, Postfach 261, D-8000 München 22 (DE)**

(72) Erfinder: **Hetz, Walter, Adam-Kraft-Strasse 17, D-8520 Erlangen (DE)**
Erfinder: **Derndinger, Walter, Gaisbühlstrasse 24, D-8520 Erlangen (DE)**

(54) **Gerät zur Ultraschallabtastung von Objekten.**

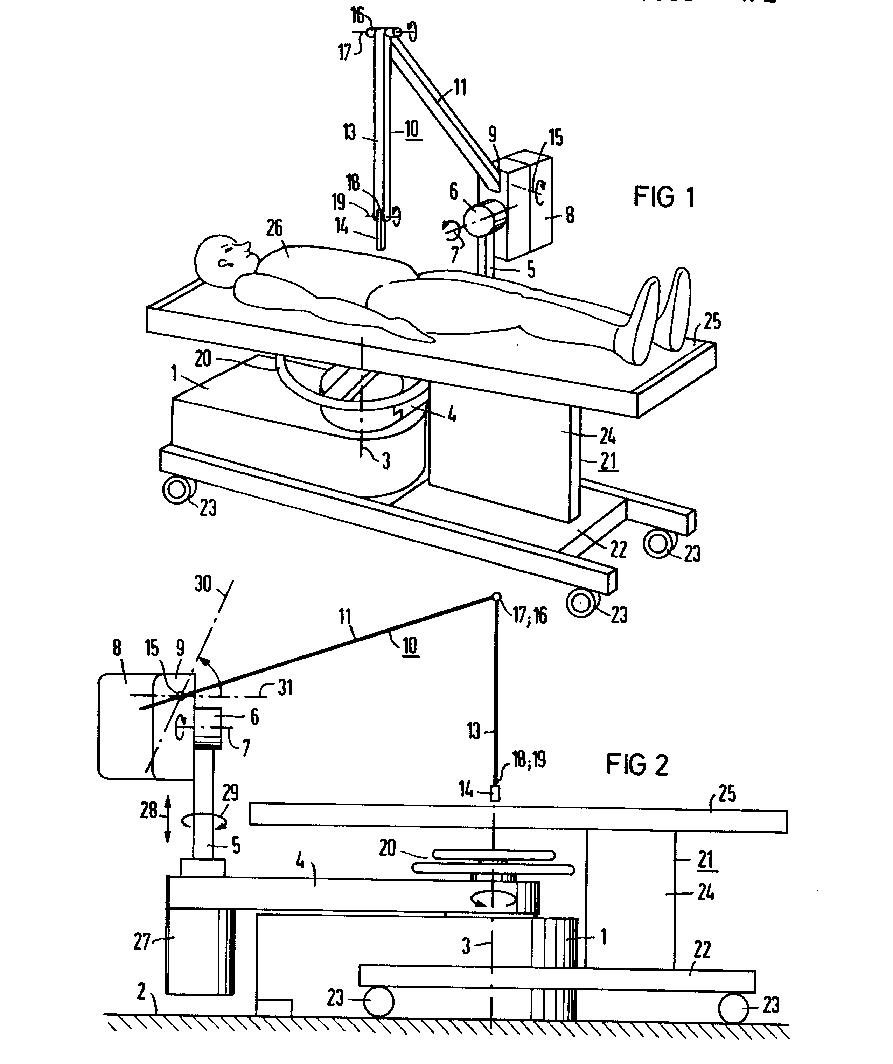

(57) Die Erfindung bezieht sich auf ein Gerät zur Ultraschallabtastung von Objekten, insbesondere des menschlichen Körpers, mit einem Ultraschall-Abtastkopf (14), der mittels Gelenkarm (10) um eine vertikale Zentralachse (3) drehbar an einem aus Basisfuß (1) und Schwenkausleger (4) gebildeten Bodenstativ befestigt ist, wobei die vertikale Zentralachse (3) in gedachter Verlängerung durch einen Objektträger (25) hindurch Isozen-trum für vom Ultraschall-Abtastkopf bei Ausrichtung des Gelenkarmes auf die Zentralachse zu legende Körperschnitte ist. Erwünscht ist der Aufbau eines solchen Gerätes, das bei der Schnittebeneneinstellung besonders einfach zu handhaben ist. Insbesondere soll die zu bewegende Gerätemasse klein sein, so daß auch eine Drehung des gesamten Abtastsystems ohne Beanspruchung einer Hilfsperson direkt am Arbeitsplatz von der Untersuchungsperson durchgeführt werden kann. Dies wird dadurch erreicht, daß an einem bodenfesten Zentralfuß (1) als Basis des Bodenstativs um eine durch diesen Zentralfuß gehende vertikale Zentralachse (3) der Schwenkausleger (4) als Träger für den Gelenkarm (10) drehbar angelenkt ist und daß der Objektträger (25), vorzugsweise beweglich verschiebbar, über dem bodenfesten Zentralfuß (1) und dem unterhalb des Trägers horizontal schwenkbaren Ausleger (4) anordbar ist (Fig. 1).

SIEMENS AKTIENGESELLSCHAFT   Unser Zeichen

Berlin und München   VPA. 78 P 5059 EUR

Gerät zur Ultraschallabtastung von Objekten

Die Erfindung bezieht sich auf ein Gerät zur Ultraschallabtastung von Objekten, insbesondere des menschlichen Körpers, mit einem Ultraschall-Abtastkopf, der mittels Gelenkarm um eine vertikale Zentralachse drehbar an einem aus Basisfuß und Schwenkausleger gebildeten Bodenstativ befestigt ist, wobei die vertikale Zentralachse in gedachter Verlängerung durch einen Objektträger hindurch Isozentrum für vom Ultraschall-Abtastkopf bei Ausrichtung des Gelenkarmes auf den Zentralachse zu legende Körperschnitte ist.

Bei bekannten Geräten dieser Art ist ein mittels Bodenrollen verschiebbarer Basisfuß der Träger des Gelenkarmes. Der Basisfuß ist wiederum mittels des Schwenkauslegers an dem dem Basisfuß abgewandten Auslegerende um eine vertikale Achse drehbar am Objektträger (Patientenliege) angelenkt. Letztere mit dem Objektträger fest verbundene Vertikalachse ist schließlich die Zentralachse, um die zur Schnittebenendrehung der komplette

Kue 5 Kof / 26.5.1978

Basisfuß zusammen mit dem Ausleger auf den Bodenrollen entlang einem Kreisbogen bewegt werden muß.

Aufgabe der Erfindung ist es, im Hinblick hierauf ein Gerät zu schaffen, das bei der Schnittebeneneinstellung einfacher zu handhaben ist. Insbesondere soll die zu bewegende Gerätemasse so klein wie möglich gehalten werden. Demgemäß sollte auch eine Drehung des gesamten Abtastsystems bewegungsfrei und bei Vermeidung jeder Verkantung ohne Beanspruchung einer Hilfsperson direkt am Arbeitsplatz (Objektträger) von der Untersuchungsperson durchführbar sein.

Die Aufgabe wird erfindungsgemäß dadurch gelöst, daß an einem bodenfesten Zentralfuß als Basis des Bodenstativs um eine durch diesen Zentralfuß gehende vertikale Zentralachse der Schwenkausleger als Träger für den Gelenkarm drehbar angelenkt ist und daß der Objektträger, vorzugsweise beweglich verschiebbar, über dem bodenfesten Zentralfuß und dem unterhalb des Trägers horizontal schwenkbaren Ausleger anordbar ist.

Beim Gerät nach der Erfindung ist also der relativ schwere Zentralfuß jetzt bodenfest und es wird nur noch die relativ leichtgewichtige Anordnung des Gelenkarmes mit Ultraschall-Abtastkopf am Schwenkausleger zusammen mit diesem gedreht. Es ist leicht einzusehen, daß die Handhabung der Drehung hierdurch stark erleichtert ist. Sie kann durchaus von der Untersuchungsperson direkt vom Arbeitsplatz her, d.h. Sitzplatz oder Standplatz am Objektträger, ohne Beanspruchung einer Hilfsperson durchgeführt werden.

Eine solche Drehung kann in bevorzugter Ausgestaltung durch einen Handantrieb, z.B. Handrad in der Zentralachse des Schwenkauslegers am bodenfesten Zentralfuß, durchgeführt werden. Ebensogut kann aber auch die Drehung motorisch durchgeführt werden; da nur verhältnismäßig geringe Massen bewegt werden müssen, können die dazu verwendeten Motoren solche mit relativ geringer Leistung sein, die wenig Raum beanspruchen und kostengünstig sind. Das Gerät nach der Erfindung erlaubt auch aufgrund seiner einfachen Handhabung das rasche Einfahren des Ultraschall-Abtastkopfes in eine erwünschte Abtastrichtung. So kann also wesentlich rascher als bisher z.B. von Längsschnittführung auf Querschnittführung umgeschaltet werden. Wird dabei zusätzlich noch in vorteilhafter Ausgestaltung der Erfindung der Schwenkausleger als Dreh-Parallelschieb-Umsetzer, z.B. in Form eines Parallelogrammarmes oder eines Rundumbandantriebes, ausgebildet oder beinhaltet der Schwenkausleger einen solchen Umsetzer, so läßt sich hierdurch die Gesamtdrehbewegung des Schwenkauslegers bei beliebigen Stellungen zur Schnittführung des Ultraschall-Abtastkopfes zum Objektträger in zueinander parallele Linearschritte umsetzen. Die Abtastung eines Objektes, z.B. des menschlichen Körpers, in parallelen, gut reproduzierbaren Schnitten wird hierdurch in optimaler Weise vereinfacht.

Weitere Vorteile und Einzelheiten der Erfindung ergeben sich aus der nachfolgenden Beschreibung eines Ausführungsbeispiels anhand der Zeichnung in Verbindung mit den Unteransprüchen.

Es zeigen:

Fig. 1 ein Ausführungsbeispiel der Erfindung in perspektivischer Ansicht,

Fig. 2 das Ausführungsbeispiel der Fig. 1 in Seitenansicht,

Fig. 3 die Ausbildung des Schwenkauslegers als
Parallelogrammarm,

Fig. 4 die Ausbildung des Schwenkauslegers als
Rundumbandantrieb,

Fig. 5 die Darstellung einer Umschaltung von
Längsabtastung auf Querabtastung in zueinander parallelen Schnittebenen.

Die Fig. 1 zeigt in perspektivischer Ansicht einen
Zentralfuß 1, der fest auf dem Boden 2 des Untersuchungsraumes steht. Er kann dazu mit dem Boden unverrückbar verbunden, z.B. an diesem angeschraubt, sein.
Ebensogut kann er allein aufgrund entsprechend hohen
Gewichtes, das ein Verschieben nur unter großer Kraftaufwendung erlaubt, praktisch unverrückbar sein. Am
Zentralfuß 1 ist in einer vertikalen Zentralachse 3
ein Schwenkausleger 4 (nur teilweise sichtbar) um die
Zentralachse drehbar angelenkt. Der Schwenkausleger 4
ist Träger einer Hubsäule 5 (ebenfalls nur teilweise
sichtbar), an deren oberem Ende mittels Drehglied 6 um
die horizontale Drehachse 7 schwenkbar ein Kasten 8
angebracht ist. Aus einer Öffnung 9 dieses Kastens
ragt ein Gelenkarm 10, der aus einem ersten Armteil 11
sowie einem zweiten Armteil 13 als Träger eines Ultraschallkopfes 14 besteht. Der Ultraschallkopf 14 ist
gleichzeitig als Handgriff zur Führung des Kopfes samt
Gelenkarm über ein Untersuchungsgebiet ausgebildet.
Der Gelenkarm 10 weist insgesamt drei Horizontaldrehgelenke auf, von denen das erste für das kastenseitige
Ende des ersten Armteiles 11 sich im Kasten 8 befindet.
Die Drehachse dieses Drehgelenkes ist in der Fig. 1
mit 15 angedeutet. Ein zweites Drehgelenk 16 mit der
Drehachse 17 verbindet die beiden Armteile 11 und 13
miteinander. Zum Verschwenken des Ultraschallkopfes 14 dient ein Drehgelenk 18 mit der Drehachse 19 am unteren Ende des zweiten Armteiles 13. Die gesamte Anordnung des Gelenkarmes 10 mit Kasten 8 und Hubsäule 5 auf dem Schwenkausleger 14 läßt sich auf einfache Weise mittels eines Handrades 20 um die Zentralachse 3 des Zentralfußes 1 drehen.

Die Ultraschall-Abtastvorrichtung der Fig. 1 dient speziell zur Abtastung des menschlichen Körpers. Aus diesem Grunde ist auch dem Zentralfuß 1 eine fahrbare Patientenliege 21 zugeordnet. Die Patientenliege besteht aus dem fahrbaren Untersatz 22 mit den Rollen 23. Auf einem Zwischenträger 24 ist schließlich das eigentliche Liegebrett 25 für einen Patienten 26 montiert. Die Patientenliege 21 ist derart über den Zentralfuß 1 schiebbar (z.B. auch durch zusätzliches Verschieben des Liegebrettes 25 relativ zum Untersatz 22), daß die vertikale Zentralachse 3 des Zentralfußes 1 in gedachter Verlängerung durch das Liegebrett 25 hindurch den Patienten 26 im Bereich zu untersuchender Körperpartien, z.B. Leber, Herz, Nieren od.dgl., durchdringt. Der Durchdringungspunkt bildet bei Ausrichtung des Gelenkarmes 10 auf die Zentralachse 3 Isozentrum für die vom Ultraschall-Abtastkopf 14 zu legenden Körperschnitte am Patienten.

In der Fig. 1 ist der Schwenkausleger 4 samt Gelenkaufbau 5, 6, 8, 10 mittels des Handrades 20 in eine solche Position geschwenkt, in der der Arm 10 samt Ultraschallkopf 14 quer zur Längsachse des Patienten 26 ausgerichtet ist. Diese Stellung ist die bevorzugte Stellung zur Durchführung von parallelen Querschnitten am Patientenkörper. Die Fig. 2 zeigt nun in Seitenansicht eine weitere wichtige Position, nämlich die Drehposition, in der der Gelenkarm 10 samt Ultraschallkopf 14 im wesentlichen in Längsrichtung des Patientenkörpers 26 ausgerichtet ist. Diese Position dient zur Legung von Längsschnitten. Selbstverständlich sind auch Schnittführungen in beliebigen anderen Winkelpositionen zwischen Quer- und Längsschnitt möglich.

In der Seitenansicht der Fig. 2 sind auch die in der perspektivischen Ansicht der Fig. 1 verdeckten Teile des Schwenkauslegers 4 bzw. auch der Hubsäule 5 besser sichtbar. Erkennbar ist auch am äußeren Ende des Schwenkauslegers 4 in Verlängerungsrichtung des Hubträgers 5 die Anordnung eines Kastens 27. In diesem Kasten befindet sich ein Antrieb zur Längsverschiebung der Säule 5 in den Pfeilrichtungen 28 (oder, falls erwünscht, auch ein Drehantrieb zum Drehen der Säule 5 in Richtung des Drehpfeiles 29). Am Kasten 8 für den Haltearm 10 sind ferner die sich bei Anschlag an den Kanten der Öffnung 9 jeweils ergebenden maximalen Auslenkpositionen des ersten Armteiles 11 des Schwenkarmes 10 mit den Kennziffern 30 bzw. 31 angedeutet. Der Gelenkarm 10 ist selbstverständlich im Armteil 11 (oder auch Armteil 13) für jede Höhen- oder auch Schwenkposition gewichtsausgeglichen.

Wird also beim Ausführungsbeispiel der Fig. 1 und 2 der zu untersuchende Patientenkörper 26 über der Zentralachse 3 gelagert und wird die Horizontalachse 7 durch entsprechendes Schwenken der Hubsäule 5 durch Schieben des Gelenkarmes 10 (oder durch Betätigung eines Drehmotors) in Höhe und Richtung auf den zu untersuchenden Ort des Patientenkörpers eingestellt, dann können mittels des Gelenkarmes 10 mit dem Schallkopf 14 Schnittebenen durch den Patientenkörper gelegt werden, die auch bei Drehung des gesamten Systems um die Zentralachse 3 oder auch bei Schwenkung des Gelenkarmes 10 mit Schallkopf 9 um die Horizontalachse 7 sich immer in der Zentralachse 3 kreuzen. Die Zentralachse ist demnach Isozentrum für sämtliche gelegten Körperschnitte.

Über die drehbare und höhenverstellbare Hubsäule 5 kann durch geeigneten Dreh-Parallelschieb-Umsetzer, z.B. durch Ausbildung des Schwenkauslegers als Parallelogrammarm oder Anordnung eines Rundumbandantriebes im Schwenkausleger, eine parallele horizontale Zwangsführung des Gelenkarmes 10 mit Schallkopf 14 erreicht werden. In diesem Falle wird die Gesamtdrehbewegung des Schwenkauslegers 4 bei beliebigen Stellungen zur Schnittführung des Ultraschall-Abtastkopfes 14 zum Objektträger 25 in zueinander parallele Linearschritte umgesetzt. Damit ist auf einfache Weise die Abtastung des Patientenkörpers 26 in parallelen, gut reproduzierbaren Schnitten ermöglicht. Die vertikale Höhenverstellung der Hubsäule erlaubt ferner auch die parallele vertikale Führung des Gelenkarmes mit Schallkopf zur Erfassung paralleler Schnittebenen bei vertikaler oder zur Horizontale geneigter Abtastung. Die Positionserfassung sämtlicher Bewegungen für die Schnittebenendarstellung an einem Sichtgerät, z.B. Oszillographenröhre, erfolgt wie üblich über Translations- bzw. Rotationsgeber bei elektronischer Verarbeitung der Informationen.

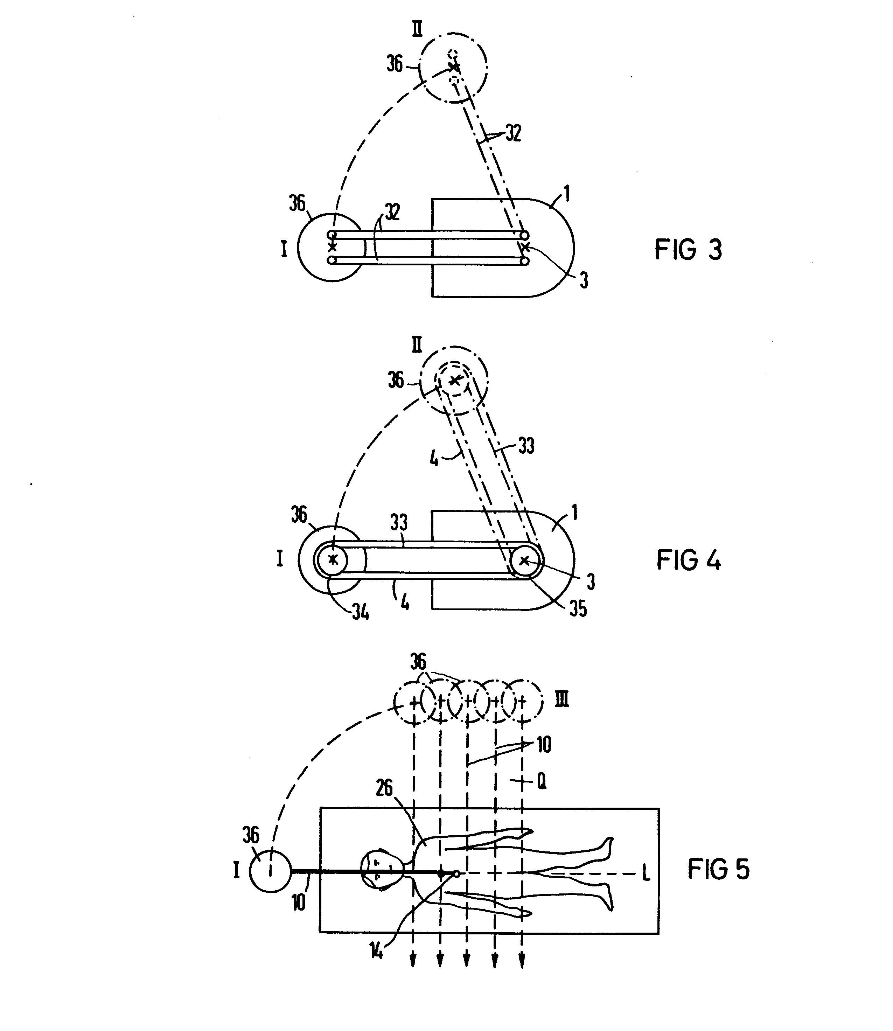

Die Fig. 3 zeigt die Ausbildung des Schwenkauslegers als Parallelogrammarm 32 in zwei Schwenkpositionen I und II.

Entsprechend zeigt die Fig. 4 eine Modifikation dahingehend, daß im Schwenkausleger 4 ein Rundumbandantrieb mit Kette oder Riemen 33 und Antriebsrollen 34 bzw. 35 angeordnet ist. Zwei Schwenkpositionen sind wiederum mit I und II angedeutet. Die Kennziffer 36 beinhaltet
Gelenkarm samt Ultraschallabtastkopf und Kasten in
schematischer Darstellung.

Die Fig. 5 zeigt schließlich in Draufsicht die Umschwenkung von Längsschnitt L aus der Position I in
Querschnittabtastung Q in den Positionen III mit Ausbildungen des Schwenkauslegers gemäß den Fig. 3 und 4.

Patentansprüche

1. Gerät zur Ultraschallabtastung von Objekten, insbesondere des menschlichen Körpers, mit einem Ultraschall-Abtastkopf, der mittels Gelenkarm um eine vertikale Zentralachse drehbar an einem aus Basisfuß und Schwenkausleger gebildeten Bodenstativ befestigt ist, wobei die vertikale Zentralachse in gedachter Verlängerung durch einen Objektträger hindurch Isozentrum für vom Ultraschall-Abtastkopf bei Ausrichtung des Gelenkarmes auf die Zentralachse zu legende Körperschnitte ist, d a d u r c h   g e k e n n z e i c h n e t , daß an einem bodenfesten Zentralfuß (1) als Basis des Bodenstativs um eine durch diesen Zentralfuß gehende vertikale Zentralachse (3) der Schwenkausleger (4) als Träger für den Gelenkarm (10) drehbar angelenkt ist und daß der Objektträger (25), vorzugsweise beweglich verschiebbar, über dem bodenfesten Zentralfuß (1) und dem unterhalb des Trägers horizontal schwenkbaren Ausleger (4) anordbar ist.

2. Gerät nach Anspruch 1, d a d u r c h   g e k e n n z e i c h n e t , daß der Schwenkausleger (4) als Dreh-Parallelschieb-Umsetzer, z.B. in Form eines Parallelogrammarmes (32) oder eines Rundumbandantriebes (33, 34, 35), ausgebildet ist oder einen solchen beinhaltet, der die Gesamtdrehbewegung des Schwenkauslegers (4) bei beliebigen Stellungen zur Schnittführung des Ultraschall-Abtastkopfes (14) zum Objektträger (21) in zueinander parallele Linearschritte umsetzt.

3. Gerät nach Anspruch 1 oder 2, d a d u r c h   g e k e n n z e i c h n e t , daß der Schwenkausleger (4) zusammen mit Gelenkarm (10) und Ultraschall-Abtastkopf (14) mittels Handantrieb, z.B. Handrad (20) in der Zentralachse, um die Zentralachse (3) drehbar ist.

4. Gerät nach einem der Ansprüche 1 bis 3, g e k e n n - z e i c h n e t   d u r c h   eine dem Gelenkarm (10) und Ultraschall-Abtastkopf (14) zugeordnete Hubsäule (5) zum Einfahren des Gelenkarmes samt Ultraschall-Abtastkopf in verschiedene Höhenpositionen oder zum Durchfahren solcher Positionen zwischen einem Anfangs- und einem Endwert.

5. Gerät nach Anspruch 4, d a d u r c h   g e k e n n - z e i c h n e t , daß die Hubsäule (5) auch um ihre vertikale Längsachse als Drehachse drehbar ist.

6. Gerät nach einem der Ansprüche 1 bis 5, g e k e n n - z e i c h n e t   d u r c h   Arretiermittel, wie z.B. Magnetbremsen, in einzelnen oder allen Dreh- oder Verschwenkgelenken zur Arretierung der Abtastanordnung (4, 5, 6, 8, 9, 10) in bestimmten Abtastpositionen bei gleichzeitiger Freigabe anderer.

7. Gerät nach einem der Ansprüche 1 bis 6, g e k e n n - z e i c h n e t   d u r c h   einen Objektträger (21), bestehend aus Untersatz (22) und relativ zum Untersatz beweglichem Objektbrett (25).

8. Gerät nach Anspruch 7, d a d u r c h   g e k e n n - z e i c h n e t , daß das Objektbrett (25) in horizontaler Ebene relativ zum Untersatz (22) beweglich ist.

FIG 1

FIG 2

FIG 3

FIG 4

FIG 5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US-A- 2924101 (SHERMAN)<br>* fig.1 v.fig.3* | 1,5,6 |
| A | US-A- 3854471 (WILD)<br>* fig.1 u.fig.2; Spalte 3, Zeilen 30-44* | 1,7 |
| A | DE-A- 2734683 (G.D.SEARLE)<br>* fig.1; Seiten 13-15* | 1 |
| A | DE-B- 1573745 (PICKER EL.)<br>* fig.1; Spalten 2-4* | 1 |
| A | DE-A- 1773587 (AUTOMATION)<br>* fig 1; Seiten 10-12* | 1 |
| A | DE-A- 1541163 (SIEMENS)<br>* fig.1; Seite 3, 3. Absatz*<br>-i- | 1 |

**KLASSIFIKATION DER ANMELDUNG (Int.Cl.3)**

A61B 10/00

G01S 15/00

G01N 29/00

**RECHERCHIERTE SACHGEBIETE (Int. Cl.2)**

A61B 10/00
G01S 9/66
G01N 29/00
29/02
29/04
A61H 23/00
A61B 6/00
6/02
6/04
6/08

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| WIEN | 10-08-1979 | LUDWIG |

EPA form 1503.1  06.78